# FASCICULE DE BREVET EUROPEEN

(11) **EP 3 595 625 B1**
(45) Date de publication et mention de la délivrance du brevet: **29.09.2021**
(21) Numéro de dépôt: 18711340.2
(22) Date de dépôt: 16.03.2018
(51) Int. Cl.: A61P 19/10, A61K 8/60, A61K 8/37, A61Q 1/14, A61K 8/04

(54) **CONCENTRE COMPRENANT AU MOINS UN LIPIDE DE MANNOSYLERYTHRITOL ET AU MOINS UN ESTER D'ACIDE GRAS ET DE POLYGLYCEROL**
KONZENTRAT MIT MINDESTENS EINEM MANNOSYLEROYTHRITOLLIPID UND MINDESTENS EINEM POLYGLYCEROL- UND FETTSÄUREESTER
CONCENTRATE COMPRISING AT LEAST ONE MANNOSYLERYTHRITOL LIPID AND AT LEAST ONE POLYGLYCEROL AND FATTY ACID ESTER

(30) Priorité: 17.03.2017 FR 1752241
(43) Date de publication de la demande: 22.01.2020
(73) Titulaire: Oleon N.V., 9940 Evergem (Ertvelde) (BE)
(72) Inventeur: PEETERS, Hilde, 3140 Keerbergen (BE)
(74) Mandataire: Santarelli
(86) Numéro de dépôt international: PCT/EP2018/056710
(87) Numéro de publication internationale: WO 2018/167285

(56) Documents cités:
- EP-A1- 3 115 355
- US-A1- 2017 071 835
- US-A1- 2017 071 837
- DATABASE WPI Week 201160 Thomson Scientific, London, GB; AN 2011-L09205 XP002774373, & JP 2011 168548 A (TOYOBO KK) 1 septembre 2011 (2011-09-01)
- DATABASE GNPD [Online] MINTEL; mars 2014 (2014-03), "Vino Moisturising Face Mask", XP002774372, Database accession no. 2340268

## Description

La présente invention se rapporte à un concentré et à des compositions le comprenant. La présente invention concerne également un procédé de préparation du concentré et des compositions selon l'invention, et leurs utilisations, en particulier celle du concentré selon l'invention en tant qu'agent épaississant, moussant et/ou nettoyant (détergent).

Les agents épaississants, moussants et/ou nettoyants trouvent une application dans de nombreux domaines. Il est notamment connu d'utiliser des agents épaississants, moussants et/ou nettoyants en cosmétique, par exemple dans des compositions démaquillantes. Il est également connu d'utiliser des agents épaississants, moussants et/ou nettoyants dans l'industrie du nettoyage, par exemple dans la préparation de produits nettoyant ou détergent tels que des compositions d'entretien ménager ou industriel, notamment des compositions de nettoyage pour surfaces dures ou des produits vaisselle.

Le cocamide diéthanolamine (ou cocamide DEA) est un agent tensioactif possédant de bonnes propriétés moussante et épaississante, usuellement utilisé dans les compositions nettoyantes comme les produits vaisselles, ou en cosmétique. Il est également courant d'utiliser des composés sulfatés, tels que le lauryl sulfate de sodium (SLS) ou le lauryl éther sulfate de sodium (SLES). Ces agents tensioactifs sulfatés possèdent de très bonnes propriétés moussante et nettoyante. Le cocamide DEA et les composés sulfatés peuvent être utilisés en association, de sorte à combiner les propriétés moussante, épaississante et nettoyante de ces composés.

Cependant, le cocamide DEA et les composés sulfatés tels que le SLS et le SLES sont considérés comme des substances à risques pour la santé humaine.

Le cocamide DEA, notamment, serait un agent carcinogène potentiel.

Le SLS et le SLES, pour leur part, sont irritants pour la peau et les yeux. Ces composés sont de plus corrosifs, et provoqueraient ainsi la détérioration des lipides et graisses composant les muscles et la peau. En outre, le SLS et le SLES sont généralement contaminés par un agent carcinogène, le 1,4-dioxane, qui est un sous-produit du processus de fabrication de ces composés sulfatés. Le document US 2017/0071837 divulgue des agents nettoyants, ou des agent nettoyants concentrés comprenant de l'eau et des bio-surfactants.

A ce jour, il existe donc un besoin pour des solutions de remplacement de ces substances à risques.

Plus particulièrement, il serait intéressant de développer des agents :
- possédant une bonne propriété tensioactive,
- possédant à la fois de bonnes propriétés épaississante, moussante et/ou nettoyante,
- permettant l'obtention de mousses stables, et
- qui seraient de moindre toxicité pour les utilisateurs.

Le travail de l'inventeur a permis de mettre en évidence qu'un concentré spécifique présentait l'ensemble des propriétés avantageuses décrites ci-avant.

L'invention concerne donc un concentré comprenant :
- au moins 20% en poids d'au moins un lipide de mannosylérythritol, par rapport au poids total du concentré, et
- au moins 30% en poids d'au moins un ester d'acide gras et de polyglycérol, par rapport au poids total du concentré,
dans lequel le ratio lipide(s) de mannosylérythritol/ester(s) d'acide(s) gras et de polyglycérol est compris dans la gamme [1 / 3 ; 3 / 1].

On notera que, dans le cadre de la présente demande, et sauf stipulation contraire, le « ratio » signifie ratio en poids et les gammes de valeurs indiquées s'entendent bornes incluses.

Par « lipide de mannosylérythritol » ou MEL, on entend un tensioactif comportant une partie hydrophile formée par le groupe mannosylérythritol, et une partie hydrophobe formée par au moins un groupe acyle.

Par MEL, on désigne plus particulièrement une molécule présentant la formule générale (I) suivante : 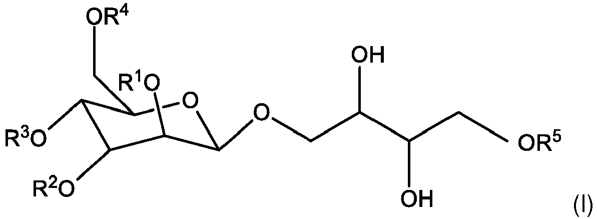 dans laquelle :
- R¹ et R², identiques ou différents, représentent un groupe acyle, comportant une chaine carbonée acyclique insaturée ou saturée,
- R³ et R⁴, identiques ou différents, représentent un groupe acétyle ou un atome d'hydrogène, et
- R⁵ représente un atome d'hydrogène ou un groupe acyle.

Parmi les MELs de formule (I) décrits ci-avant, on peut distinguer les « MELs di-acylés » des « MELs tri-acylés », selon la nature du groupe présent en R⁵. On notera que selon cette terminologie, les groupements acétyles pouvant être présents en R³ et R⁴ ne sont pas comptabilisés dans les groupements acyles.

Par MEL tri-acylé, on désigne une molécule de formule (I) dans laquelle :
- R¹ et R², identiques ou différents, représentent un groupe acyle, comportant une chaine carbonée acyclique insaturée ou saturée,
- R³ et R⁴, identiques ou différents, représentent un groupe acétyle ou un atome d'hydrogène, et
- R⁵ représente un groupe acyle.

Par MEL di-acylé, on désigne une molécule de formule (I) dans laquelle :
- R¹ et R², identiques ou différents, représentent un groupe acyle, comportant une chaine carbonée acyclique insaturée ou saturée,
- R³ et R⁴, identiques ou différents, représentent un groupe acétyle ou un atome d'hydrogène, et
- R⁵ représente un atome d'hydrogène.

Un MEL di-acylé est donc représenté par la formule (II) suivante :

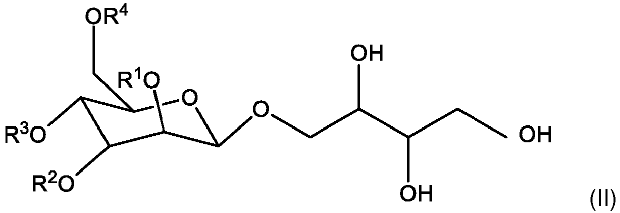

Avantageusement, le au moins un MEL compris dans le concentré selon l'invention est di-acylé.

Deux stéréoisomères de MEL di-acylé de formule (II) sont connus et représentés dans les formules (III) et (IV) ci-après : 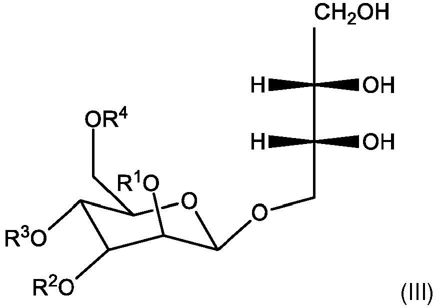 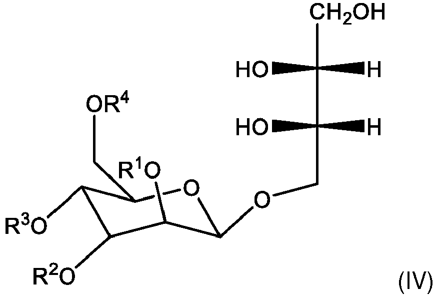 dans lesquelles, R¹, R², R³, R⁴ sont identiques à ceux indiqués en Formule (II). Avantageusement, un MEL di-acylé est une molécule de formule (III).

Les formules (I) à (IV) ci-avant peuvent représenter plusieurs molécules, chaque molécule étant donc un MEL. Par « MELs », on désigne au moins deux molécules de formules (I), (II), (III) ou (IV) différentes de par leur substitution (groupes acyles, acétyles) ou par leur stéréoisomérie, plus particulièrement, au moins deux molécules de formules (III) différentes.

Par ailleurs, les MELs sont généralement classés en quatre classes de molécules, notées de A à D, selon leur degré d'acétylation en R³ et R⁴. La classe des MELs-A comporte des molécules de formule (I) présentant deux groupes acétyles en R³ et R⁴. La classe des MELs-B et la classe des MELs-C comportent des molécules de formule (I) présentant un seul groupe acétyle en R⁴ et R³ respectivement. Enfin, la classe des MELs-D comporte des molécules de formule (I) ne présentant pas de groupe acétyle (R³= R⁴=H).

Outre de par leur degré d'acétylation, les MELs peuvent varier dans leur structure, de par la nature des acides gras qui composent leur partie hydrophobe. Cette variation est généralement fonction du procédé mis en oeuvre pour l'obtention des MELs.

Les MELs sont généralement obtenus par des procédés mettant en œuvre la culture de champignons, et plus particulièrement de levures.

Avantageusement, le(s) MEL(s) visé(s) par la présente demande sont obtenus par un procédé de fermentation, comprenant les étapes suivantes :
- la culture d'une souche de champignon et plus particulièrement d'une souche de levure en présence d'une source de carbone pour obtenir des MELs; et
- la récupération des MELs ainsi obtenus.

Les souches à partir desquelles il est possible d'obtenir des MELs sont bien connues de l'homme du métier. A titre d'exemple, il est connu d'utiliser des souches de la famille des Basidiomycètes, de préférence du genre Pseudozyma, telle que *Pseudozyma antarctica, Pseudozyma parantartica, Pseudozyma aphidis, Pseudozyma rugulosa, Pseudozyma graminicola, Pseudozyma siamensis, Pseudozyma hubeiensis, Pseudozyma tsukubaensis, Pseudozyma crassa,* ou du genre Ustilago, telle que *Ustilago maydis, Ustilago cynodontis* et *Ustilago scitaminea.*

En général, selon la souche, une classe de MELs (MELs-A, MELs-B, MELs-C ou MELs-D), est produite majoritairement, voire exclusivement par rapport aux autres classes de MEL. A titre d'exemple, *Pseudozyma antarctica, Pseudozyma aphidis, Pseudozyma rugulosa* et *Pseudozyma parantarctica* produisent en majorité des MELs-A de formule (III). *Pseudozyma graminicola, Pseudozyma siamensis, Pseudozyma hubeiensis* produisent en majorité des MELs-C de formule (III). *Pseudozyma tsukubaensis* produit en majorité des MELs-B de formule (IV) et *Pseudozyma crassa* produit en majorité des MELs- A de formule (IV).

Avantageusement, les MELs sont obtenus par un procédé de fermentation mettant en oeuvre une souche produisant des MELs de formule (III).

Plus particulièrement, les MELs sont obtenus par un procédé de fermentation mettant en oeuvre une souche choisie parmi *Pseudozyma aphidis, Pseudozyma rugulosa, Pseudozyma antarctica* ou *Pseudozyma parantarctica,* préférentiellement parmi *Pseudozyma aphidis, Pseudozyma antarctica* ou *Pseudozyma parantarctica,* plus préférentiellement, la souche est *Pseudozyma aphidis.*

Le substrat carboné est typiquement un glycérol, un n-alcane ou une huile, en particulier d'origine renouvelable.

Toute huile, composée de triglycérides et liquide à la température du procédé de fermentation, peut être utilisée comme substrat carboné.

Préférentiellement, l'huile renouvelable est une huile végétale ou animale, plus préférentiellement, une huile végétale. En particulier, l'huile végétale est choisie parmi le groupe constitué par une huile de soja, une huile de tournesol, une huile d'olive et une huile de colza. Plus particulièrement, l'huile végétale est une huile de soja ou une huile de colza, plus particulièrement encore, une huile de colza.

Ces huiles renouvelables sont particulièrement riches en groupes acyles comportant une chaine carbonée à 18 atomes de carbone, tels que les groupes acyles issus de l'acide oléique, linoléique et/ou linolénique.

Le procédé de fermentation dure généralement au moins 3 jours, préférentiellement au moins 7 jours.

Selon un mode de réalisation préférentiel, les MELs sont obtenus par un procédé de fermentation mettant en oeuvre :
- une souche du genre Pseudozyma, préférentiellement *Pseudozyma antartica, Pseudozyma parantarctica,* ou *Pseudozyma aphidis,*
- une huile végétale, préférentiellement une huile de colza ou une huile de soja, en tant que substrat carboné.

Une telle souche est usuellement cultivée en réacteur dans un milieu comportant du glucose, de l'eau et/ou des sels (tel que le sulfate de magnésium, le phosphate de monopotassium, le nitrate de sodium, et/ou le nitrate d'ammonium). Ce milieu de culture est également mis en oeuvre dans le procédé de fermentation. En effet, d'une manière générale, le milieu de fermentation du procédé de fermentation, comporte un milieu de culture et le substrat carboné.

Avantageusement, les différents composants du milieu (glucose et souche en particulier) sont stérilisés séparément avant introduction dans le réacteur.

La température du milieu est de préférence comprise entre 20°C et 40°C, plus préférentiellement entre 25°C et 35°C.

Le brut réactionnel obtenu à l'issue du procédé de fermentation, est ce qui est appelé dans la présente demande, le brut de fermentation.

Le brut de fermentation comporte généralement au moins deux MELs di-acylés, au moins du substrat carboné résiduel et/ou un sous-produit du substrat carboné, la souche et de l'eau, le sous-produit du substrat carboné résultant de la fermentation.

L'étape de récupération des MELs a pour objectif de séparer un/des MEL(s) d'un ou de plusieurs des autres composants du brut de fermentation, tels que du substrat carboné résiduel et/ou un sous-produit du substrat carboné, une souche, et/ou de l'eau.

Selon le mode de réalisation préférentiel ci-avant, le brut de fermentation comporte au moins deux MELs di-acylés, au moins un triglycéride et/ou au moins un acide gras, de l'eau et une souche du genre Pseudozyma.

En effet, lorsque le substrat carboné est une huile d'origine renouvelable, un sous-produit du substrat carboné est un acide gras. De plus, une huile végétale étant principalement (plus de 90% en poids) constituée de triglycérides, l'huile végétale résiduelle est donc composée d'au moins un triglycéride.

La séparation d'un ou des MEL(s) d'un ou de plusieurs des autres composants du brut de fermentation peut se faire par toute méthode de séparation connue de l'homme du métier.

Avantageusement, la séparation d'un ou des MEL(s) d'un ou de plusieurs des autres composants peut comprendre une ou plusieurs des méthodes suivantes :
- décantation,
- centrifugation,
- filtration,
- évaporation,
- extraction liquide/liquide,
- passage sur un substrat minéral ou une résine.

En particulier :
- la souche peut être séparée par décantation, filtration, et/ou centrifugation ;
- l'eau peut être séparée par décantation, évaporation, centrifugation, et/ou passage sur un substrat minéral qui est un adsorbant;
- les acides gras et les triglycérides peuvent être séparés par extraction liquide/liquide et/ou par passage sur un substrat minéral ou une résine.

Les MELs récupérés peuvent donc comporter :
- au moins un triglycéride et/ou au moins un acide gras, et
- optionnellement, une souche.

Par « acide gras », on entend un acide gras libre et/ou sous forme de sel.

La quantité d'acide(s) gras et/ou de triglycéride(s) présente dans les MELs récupérés peut être comprise entre 0,5 et 60% en poids, de préférence entre 1 et 50% en poids, par rapport au poids total de MELs récupérés.

Avantageusement, le ou les acide(s) gras comporte(nt) une chaine carbonée comportant entre 8 et 24 atomes de carbone, de préférence, entre 8 et 20 atomes de carbone.

Avantageusement, le ou les triglycéride(s) comporte(nt) des groupes acyles dont la chaine carbonée acyclique, saturée ou insaturée, comporte entre 8 et 24 atomes de carbones, de préférence entre 16 et 18 atomes de carbone. Plus particulièrement, la chaine carbonée est linéaire et ne comporte que des atomes de carbone et d'hydrogène, éventuellement substituée par une fonction hydroxyle (OH).

Les MELs récupérés peuvent donc se trouver sous une forme plus ou moins purifiée, c'est-à-dire en mélange avec d'autres composants du milieu de fermentation.

Plus particulièrement, dans la présente demande, et en particulier dans les exemples, lorsque les MELs récupérés, sont en mélange avec au moins un acide gras et/ou au moins un triglycéride, optionnellement de l'eau et/ou une souche, ce mélange est appelé « mélange de MELs».

Un premier mélange de MELs est un brut de fermentation, c'est-à-dire au moins deux MELs di-acylés avec les autres composants du brut de fermentation.

Le brut de fermentation peut faire l'objet d'une ou plusieurs méthodes de séparation, conduisant à d'autres mélanges de MELs préférés présentant les caractéristiques suivantes :
- une teneur en MELs supérieure ou égale à 30% en poids, préférentiellement supérieure ou égale à 40% en poids, plus préférentiellement supérieure ou égale à 50% en poids ;
- une teneur en autres composants (dont acide(s) gras, triglycéride(s), eau, et/ou souche) inférieure ou égale à 70% en poids, préférentiellement inférieure ou égale à 60% en poids, plus préférentiellement inférieure ou égale à 50% en poids ;
les pourcentages en poids étant donnés par rapport au poids du mélange de MELs.

Plus particulièrement, selon la ou les méthode(s) de séparation telle(s) que décrite(s) ci-avant, des mélanges de MELs plus ou moins concentrés en MELs peuvent être obtenus.

Selon un premier mode de réalisation, le mélange de MELs présente les caractéristiques suivantes :
- une teneur en MELs supérieure ou égale à 55% en poids ;
- une teneur en autres composants (dont acide(s) gras, triglycéride(s), eau, et/ou souche) inférieure ou égale à 45% en poids ;
les pourcentages en poids étant donnés par rapport au poids du mélange de MELs.

Avantageusement, dans ce premier mode de réalisation, la teneur en eau et/ou en souche est inférieure ou égale à 10% en poids, préférentiellement inférieur ou égale à 5% en poids, par rapport au poids du mélange de MELs.

Selon un deuxième mode de réalisation, lequel est particulièrement préféré, le mélange de MELs présente les caractéristiques suivantes :
- une teneur en MELs supérieure ou égale à 90% en poids, préférentiellement supérieure ou égale à 95% en poids, plus préférentiellement supérieure ou égale à 98% en poids ;
- une teneur en autre composants (dont acide(s) gras, triglycéride(s), eau, et/ou souche) inférieure ou égale à 10% en poids, préférentiellement inférieure ou égale à 5% en poids, plus préférentiellement inférieure ou égale à 2% en poids ;
les pourcentages en poids étant donnés par rapport au poids du mélange de MELs.

Avantageusement, dans ce deuxième mode de réalisation, la teneur en eau et/ou en souche est inférieure ou égale à 2% en poids, par rapport au poids du mélange de MELs.

Un tel mélange de MELs peut, par exemple, être obtenu à l'aide d'un procédé de fermentation tel que décrit ci-avant, comprenant plusieurs étapes de séparation telles que décrites ci-avant, ces étapes de séparation incluant préférentiellement une extraction liquide/liquide et/ou un passage sur un substrat minéral.

Le passage sur un substrat minéral peut être une chromatographie, telle qu'une chromatographie d'adsorption sur colonne de silice, réalisée à l'aide de solvants adaptés. De tels solvants sont connus de l'homme du métier.

Des exemples de mélanges de MELs et de leur procédé d'obtention sont également décrits dans la publication suivante : "Downstream processing of mannosylerythritol lipids produced by Pseudozyma aphidis" ; Rau et al.; European Journal of Lipids Science and Technology (2005), 107, 373-380.

Avantageusement, le(s) MEL(s) récupéré(s) à l'issue du procédé de fermentation décrit ci-avant est/sont di-acylé(s).

Lorsque le(s) MELs récupéré(s) à l'issue du procédé de fermentation est/sont un/des MEL(s) di-acylé(s), il est possible de mettre en oeuvre une étape subséquente de production de MELs tri-acylés à partir du/des MEL(s) di-acylé(s) (ou d'un mélange de MELs di-acylés) récupéré(s).

Cette étape subséquente comprend avantageusement :
- la dissolution du/des MEL(s) di-acylé(s) dans un solvant organique en présence d'une enzyme ; et
- l'ajout d'au moins une huile végétale, d'au moins un acide gras d'origine végétale ou d'au moins un ester d'acide gras d'origine végétale ;
dans des conditions permettant soit une réaction de transestérification entre le(s) MEL(s) di-acylé(s) et les triglycérides présents dans l'huile végétale ou l'ester d'acide gras d'origine végétale, soit une réaction d'estérification entre le(s) MEL(s) di-acylé(s) et l'acide gras d'origine végétale, permettant ainsi la production de MEL(s) tri-acylé(s).

Avantageusement, le solvant organique est choisi parmi le méthanol, l'éthanol, le propanol, le butanol, l'acétone, le propanone, le butanone, le pentane-2-one, le 1,2-éthanediol, le 2,3-butanediol, le dioxane, l'acétonitrile, le 2-méthyl-butane-2-ol, le tert-butanol, le 2-méthylpropanol, le 4-hydroxy-2-méthyl pentanone, le tétrahydrofuranne, l'hexane, le diméthylformamide (DMF), le diméthylsulfoxide (DMSO) et/ou la pyridine.

Préférentiellement, l'huile végétale est choisie parmi le groupe constitué par une huile de soja, une huile de tournesol, une huile d'olive et une huile de colza. Plus particulièrement, l'huile végétale est une huile de soja ou une huile de colza, plus particulièrement encore, une huile de colza.

Avantageusement, l'acide gras d'origine végétale ou l'ester d'acide gras d'origine végétale provient d'une huile de soja, d'une huile de tournesol, d'une huile d'olive ou d'une huile de colza. Plus particulièrement, l'acide gras d'origine végétale ou l'ester d'acide gras d'origine végétale provient d'une huile de soja ou d'une huile de colza, plus particulièrement encore, d'une huile de colza.

Ces huiles végétales sont particulièrement riches en groupes acyles comportant une chaine carbonée à 18 atomes de carbone, tels qu'en groupes acyles issus de l'acide oléique, linoléique et/ou linolénique.

L'enzyme peut être sélectionnée parmi les lipases, les protéases, et/ou les estérases, de préférence parmi les lipases et/ou estérases, encore plus préférentiellement parmi les lipases.

Avantageusement, la réaction d'estérification ou de transestérification est réalisée pendant environ 12 à 72h à une température proche (+/- 10°C) de la température optimale d'activité de l'enzyme, de préférence pendant environ 24 à 48h à une température comprise entre 20 et 30°C, plus préférentiellement à 25°C.

Le(s) MEL(s) tri-acylé(s) peu(ven)t ensuite être récupéré(s) à partir du milieu réactionnel, par des méthodes de séparation connues de l'homme du métier.

Parmi ces méthodes de séparation figurent par exemple, la chromatographie, telle que la chromatographie par absorption sur colonne de silice.

Le concentré selon l'invention comporte également au moins 30% en poids d'au moins un ester d'acide gras et de polyglycérol, par rapport au poids total du concentré.

De préférence, le concentré selon l'invention comprend un ester d'acide gras et de polyglycérol.

Avantageusement, l'acide gras compris dans l'ester d'acide gras et de polyglycérol comporte une chaine carbonée comportant entre 6 et 18 atomes.

L'ester d'acide gras et de polyglycérol étant destiné à être solubilisé dans l'eau, celui-ci est hydophyle, avantageusement de HLB supérieure ou égale à 9, préférentiellement supérieure ou égale à 10, plus préférentiellement supérieure ou égale à 12.

On entend par HLB (Hydrophilic-Lipophilic Balance) l'équilibre entre la dimension et la force du groupe hydrophile et la dimension et la force du groupe lipophile de l'agent tensioactif. La valeur HLB selon GRIFFIN est définie dans J. Soc. Cosm. Chem. 1954 (volume 5), pages 249-256.

A titre d'exemple d'esters d'acide gras et de polygycérol de HLB supérieure ou égale à 9, on peut citer l'isostéarate de polyglycérol-6, l'isostéarate de polyglycérol-10, le diisostéarate de polyglycérol-10, le laurate de polyglycérol-6, le myristate de polyglycérol-6, le stéarate de polyglycérol-6, l'oléate de polyglycérol-6, l'oléate de polyglycérol-10, le caprylate de polyglycérol-10, le caprylate de polyglycérol-6, le caprate de polyglycérol-4, le laurate de polyglycérol-4, le laurate de polyglycérol-10.

De préférence, le polyglycérol compris dans l'ester d'acide gras et de polyglycérol comprend entre 2 et 12, de préférence entre 2 et 10, plus préférentiellement entre 3 et 6 unités de glycérol.

Avantageusement, le polyglycérol de l'ester d'acide gras et de polyglycérol est un polyglycérol-4, polyglycérol-6 ou polyglycérol-10. Le nombre entier suivant le polyglycérol (ou PG) représente le nombre d'unité de glycérol formant le polyglycérol.

Préférentiellement, l'ester d'acide gras et de polyglycérol est un monoester d'acide gras et de polyglycérol ou un diester d'acide gras et de polyglycérol, plus préférentiellement un monoester d'acide gras et de polyglycérol.

Le concentré selon l'invention possède une bonne propriété épaississante. Par « propriété épaississante », on entend que le concentré selon l'invention augmente la viscosité de l'eau. En d'autres termes, une composition comprenant un concentré selon l'invention et de l'eau aura une viscosité supérieure à celle de l'eau seule, avantageusement d'au moins 30 mPa.s, de préférence d'au moins 80 mPa.s, plus préférentiellement d'au moins 200 mPa.s, encore plus préférentiellement d'au moins 300 mPa.s.

En outre, le concentré selon l'invention permet de donner à l'eau l'aspect d'un gel, c'est-à-dire qu'une composition comprenant un concentré selon l'invention et de l'eau présentera l'aspect d'un gel.

Par « aspect gel », on vise la rhéologie typique d'un gel. En particulier, la viscosité initiale d'une composition comprenant un concentré selon l'invention et de l'eau diminuera si elle est soumise à un frottement, et reviendra à sa valeur initiale suite à l'arrêt du frottement.

Ces caractéristiques du concentré selon l'invention sont plus amplement décrites dans l'Exemple 2 ci-après.

Par ailleurs, le concentré selon l'invention a une bonne propriété moussante. On entend ici qu'un concentré selon l'invention, lorsqu'il est mis au contact d'eau, permet la formation d'une mousse à la surface de la composition obtenue.

De préférence, le concentré selon l'invention permet la formation d'une mousse dans des conditions semblables à celles décrites dans la norme ASTM D892.

Dans la présente demande, toute référence à une norme est une référence à la norme en vigueur à la date de dépôt.

Avantageusement, le volume de la mousse formée à la surface d'une composition comprenant un concentré selon l'invention et de l'eau est supérieur à 200 mL, de préférence supérieur à 400 mL, encore plus préférentiellement supérieur à 600 mL. Un tel volume varie en fonction de la dureté de l'eau.

Par ailleurs, la mousse formée à la surface d'une composition comprenant un concentré selon l'invention est stable. Par « stable », on entend que le volume de mousse formée ne diminue pas ou diminue très peu au cours du temps, c'est-à-dire de moins de 50 mL en 10 min, préférentiellement de moins de 25 mL en 10 min.

La propriété moussante du concentré selon l'invention ainsi que la stabilité d'une mousse formée à la surface d'une composition comprenant le concentré selon l'invention, sont plus amplement décrites dans l'Exemple 3.

En outre, le concentré selon l'invention a une bonne propriété nettoyante. Avantageusement, le concentré selon l'invention comprend au moins 30% en poids, optionnellement au moins 45% en poids d'au moins un MEL, sur le poids total du concentré.

Avantageusement, au moins 80% en poids du concentré selon l'invention est constitué de lipide(s) de mannosylérythritol et d'ester(s) d'acide(s) gras et de polyglycérol.

De préférence, dans le concentré selon l'invention, le ratio en poids lipide(s) de mannosylérythritol/ester(s) d'acide(s) gras et de polyglycérol est compris dans la gamme [1 / 2 ; 2 / 1].

Un tel ratio permet encore d'améliorer la propriété épaississante du concentré selon l'invention.

De préférence, le au moins un ester d'acide gras et de polyglycérol compris dans le concentré selon l'invention est un ester d'acide caprique et/ou caprylique et de polyglycérol.

Un ester d'acide caprique et/ou caprylique et de polyglycérol est également appelé polyglycerol caprate et/ou caprylate, ou polyglyceryl caprate et/ou caprylate.

De préférence, le au moins un ester est un ester d'acide caprique et de polyglycérol.

Alternativement, le au moins un ester d'acide gras et de polyglycérol est un ester d'acide gras comportant 18 atomes de carbone et de polyglycérol, de préférence un ester d'acide oléique ou isostéarique et de polyglycérol.

Avantageusement, le concentré selon l'invention comprend au moins deux MELs, en particulier au moins deux MELs issus de classes différentes choisies parmi le groupe constitué par les MELs-A, MELs-B, MELs-C et MELs-D.

Selon un premier mode avantageux de réalisation, le concentré selon l'invention comporte des MELs-A, MELs-B, MELs-C et optionnellement des MELs-D, plus préférentiellement des MELs-A, MELs-B, MELs-C et des MELs-D.

Avantageusement, le concentré selon l'invention comporte des MELs-A et MELs-B à une teneur comprise entre 50% à 95% en poids, de préférence 60% à 85% en poids, les pourcentages en poids étant indiqués par rapport au poids de la quantité totale de MELs.

Avantageusement, le concentré selon l'invention comporte du ou des MELs-C à une teneur supérieure ou égale à 5% en poids, préférentiellement supérieure à 10% en poids, les pourcentages en poids étant indiqués par rapport au poids de la quantité totale de MELs.

Plus particulièrement, le concentré selon l'invention comporte des MELs-A et MELs-B à une teneur comprise entre 60% et 80% en poids et des MELs-C à une teneur supérieure ou égale à 15% en poids, les pourcentages en poids étant indiqués par rapport au poids de la quantité totale de MELs.

Selon un deuxième mode avantageux de réalisation, le concentré selon l'invention comporte des MELs-D à une teneur comprise entre 75% et 100% en poids, de préférence entre 90% et 100% en poids, les pourcentages en poids étant indiqués par rapport au poids de la quantité totale de MELs.

Les MELs-D peuvent être obtenus par désacétylation des MELs-A, MELs-B et MELs-C. Un exemple de réaction de désacétylation des MELs-A, MELs-B et MELs-C utilisant une enzyme hydrolysante est décrite dans la publication suivante : "Enzymatic synthesis of a novel glycolipid biosurfactant, mannosylerythritol lipid-D and its aqueous phase behavior " ; Fukuoka et al.; Carbohydrate Research (2011), 346, 266-271.

Avantageusement, le concentré selon l'invention comprend en outre au moins un ester d'acide gras et de glycérol.

De préférence, le concentré selon l'invention comprend un ester d'acide gras et de glycérol.

Selon un premier mode de réalisation du concentré selon l'invention, celui-ci comporte ou consiste en :
- au moins 20% en poids d'au moins un lipide de mannosylérythritol, par rapport au poids total du concentré, et
- au moins 30% en poids d'au moins un ester d'acide gras et de polyglycérol, par rapport au poids total du concentré,
dans lequel le ratio en poids lipide(s) de mannosylérythritol/ester(s) d'acide(s) gras et de polyglycérol est compris dans la gamme [1 / 3 ; 3 / 1].

Selon un second mode de réalisation du concentré selon l'invention, celui-ci comporte ou consiste en :
- au moins 20% en poids d'au moins un lipide de mannosylérythritol, par rapport au poids total du concentré,
- au moins 30% en poids d'au moins un ester d'acide gras et de polyglycérol, par rapport au poids total du concentré, et
- au moins un ester d'acide gras et de glycérol,
dans lequel le ratio en poids lipide(s) de mannosylérythritol/ester(s) d'acide(s) gras et de polyglycérol est compris dans la gamme [1 / 3 ; 3 / 1].

Le au moins un ester d'acide gras et de polyglycérol mis en oeuvre dans ces deux modes de réalisation est avantageusement un ester d'acide caprique et/ou caprylique et de polyglycérol, de préférence un ester d'acide caprique et de polyglycérol.

Avantageusement, dans le concentré selon l'invention, la quantité d'ester(s) d'acide(s) gras et de glycérol est comprise entre 1,5 et 4,5% en poids, de préférence entre 2 et 4% en poids, sur le poids total du concentré.

Les caractéristiques préférées du concentré selon l'invention décrites ci-avant s'appliquent à l'ensemble de ces modes de réalisation.

De préférence, le au moins un ester d'acide gras et de glycérol compris dans le concentré selon l'invention est un ester d'acide caprylique et de glycérol.

Lorsqu'il est ajouté à de l'eau, l'ester d'acide caprylique et de glycérol provoque un effet de flou lumineux dans l'eau. Cet effet est très intéressant pour le développement de produits cosmétiques, tel que ceux pour lesquels on souhaite obtenir une texture type lait.

Une composition, telle qu'une composition cosmétique, préparée à partir d'un concentré selon l'invention comprenant ester d'acide caprylique et de glycérol présentera des propriétés avantageuses, telles qu'un toucher agréable sans film huileux et une sensation de peau nourrie chez l'utilisateur.

Les esters d'acide caprylique et de glycérol possèdent également de bonnes propriétés antimicrobiennes.

L'invention concerne également un procédé de préparation d'un concentré selon l'invention, comprenant une étape de mélangeage d'au moins 20% en poids d'au moins un lipide de mannosylérythritol, par rapport au poids total du concentré, et d'au moins 30% en poids d'au moins un ester d'acide gras et de polyglycérol, par rapport au poids total du concentré, dans lequel le ratio en poids lipide(s) de mannosylérythritol/ester(s) d'acide(s) gras et de polyglycérol est compris dans la gamme [1 / 3 ; 3 / 1].

Avantageusement, le mélange est réalisé à une température comprise entre 40 et 60 °C, de préférence 60 °C.

Avantageusement, les composants mis en oeuvre dans le procédé de préparation d'un concentré selon l'invention présente une ou plusieurs des caractéristiques préférées décrites ci-avant.

L'invention concerne par ailleurs une composition comprenant un concentré selon l'invention, et de l'eau. Plus particulièrement, la composition selon l'invention comporte :
- au moins un MEL,
- au moins un ester d'acide gras et de polyglycérol de HLB supérieure ou égale à 9, et
- de l'eau,
dans laquelle le ratio en poids lipide(s) de mannosylérythritol/ester(s) d'acide(s) gras et de polyglycérol de HLB supérieure ou égale à 9 est compris dans la gamme [1 / 3 ; 3 / 1].

Le au moins un MEL et le au moins ester d'acide gras et de polyglycérol sont tels que décrits ci-avant, y inclus les modes avantageux et préférentiels.

La composition selon l'invention a une bonne propriété nettoyante, et plus particulièrement démaquillante. Cela est plus amplement décrit dans l'Exemple 6 ci-après.

Selon un premier mode de réalisation, la quantité de concentré ou la quantité totale de MEL(s) et d'ester(s) d'acide(s) gras et de polyglycérol de HLB supérieure ou égale à 9 dans la composition selon l'invention est comprise entre 3% et 75% en poids, sur le poids total de la composition.

Par quantité totale de MEL(s) et d'ester(s) d'acide(s) gras et de polyglycérol de HLB supérieure ou égale à 9, on entend la quantité totale en poids de molécules de MEL(s) et de molécules d'ester(s) d'acide(s) gras et de polyglycérol.

Selon ce premier mode de réalisation de la composition selon l'invention, celle-ci a une viscosité bien supérieure à celle de l'eau, et présente l'aspect d'un gel.

Selon un mode particulier de ce premier mode de réalisation, la composition selon l'invention comprend un concentré selon l'invention qui ne comporte pas d'ester d'acide gras et de glycérol. Dans ce mode particulier, lorsque la quantité de concentré ou la quantité totale de MEL(s) et d'ester(s) d'acide(s) gras et de polyglycérol de HLB supérieure ou égale à 9, comprise dans la composition selon l'invention est faible, c'est-à-dire comprise entre 3 et 7% en poids sur le poids total de la composition, il est préférable que le concentré ou la quantité totale de MEL(s) et d'ester(s) d'acide(s) gras et de polyglycérol de HLB supérieure ou égale à 9, comprenne au moins 50% en poids d'au moins un MEL, sur le poids total de concentré ou de la quantité totale de MEL(s) et d'ester(s) d'acide(s) gras et de polyglycérol de HLB supérieure ou égale à 9, respectivement. Une telle teneur en MEL permet d'obtenir une augmentation notable de la viscosité de l'eau.

Selon un mode particulier alternatif de ce premier mode de réalisation, la composition selon l'invention comporte au moins un ester d'acide gras et de glycérol. Dans ce mode particulier, une augmentation notable de la viscosité de l'eau est obtenue même à une teneur d'au moins 20% en poids de MEL(s) sur le poids total de concentré ou de la quantité totale de MEL(s) et d'ester(s) d'acide(s) gras et de polyglycérol de HLB supérieure ou égale à 9.

Selon ce premier mode de réalisation, la quantité de concentré ou la quantité totale de MEL(s) et d'ester(s) d'acide(s) gras et de polyglycérol de HLB supérieure ou égale à 9, dans la composition selon l'invention est avantageusement comprise entre 5 et 50% en poids, de préférence entre 5 et 35% en poids, sur le poids total de la composition.

Des teneurs croissantes en concentré permettent d'augmenter la viscosité de la composition selon l'invention. L'homme du métier est en mesure de définir ou d'adapter la quantité de concentré dans la composition permettant d'obtenir une viscosité souhaitée.

Selon ce mode de réalisation, la composition selon l'invention possède avantageusement une viscosité dynamique supérieure ou égale à 40 mPa.s, de préférence supérieure ou égale à 200 mPa.s, plus préférentiellement supérieure ou égale à 400 mPa.s, encore plus préférentiellement supérieure ou égale à 500 mPa.s.

Selon un second mode de réalisation, la quantité de concentré dans la composition selon l'invention, exprimée en pourcentage en poids sur le poids total de la composition, est comprise dans la gamme [0,05 ; 3[.

Selon ce second mode de réalisation de la composition selon l'invention, celle-ci n'a pas une viscosité bien supérieure à celle de l'eau, et se présente sous la forme d'une solution.

Selon ce second mode de réalisation, la quantité de concentré est avantageusement comprise entre 0,1 et 2% en poids, de préférence entre 0,15 et 1,5% en poids, sur le poids total de la composition.

L'invention concerne également un procédé de préparation d'une composition selon l'invention, comprenant une étape de mélangeage d'un concentré selon l'invention avec de l'eau.

Selon un premier mode de réalisation du procédé de préparation d'une composition selon l'invention, le au moins un MEL est mélangé à au moins un ester d'acide gras et de polyglycérol de HLB supérieure ou égale à 9, préalablement au mélangeage avec de l'eau.

Selon un second mode de réalisation du procédé de préparation d'une composition selon l'invention, le au moins un MEL est mélangé à l'eau indépendamment d'un ester d'acide gras et de polyglycérol.

Avantageusement, l'étape de mélangeage est réalisée sous agitation.

L'invention concerne également l'utilisation d'un concentré selon l'invention, en tant qu'agent épaississant, moussant et/ou nettoyant.

Le concentré selon l'invention peut être utilisé dans tout type d'applications dans lesquelles il est usuel d'utiliser un agent épaississant, moussant et/ou nettoyant.

Ce concentré peut être utilisé en tant qu'agent épaississant, moussant et/ou nettoyant dans la préparation de produits nettoyant ou détergent tels que des compositions d'entretien ménager ou industriel, notamment des compositions de nettoyage pour surfaces dures ou des produits vaisselle.

Ce concentré peut également être utilisé dans des produits de nettoyage dans le domaine pétrolier.

Ce concentré peut enfin être utilisé en tant qu'agent épaississant, moussant et/ou nettoyant en cosmétique ou dans des produits d'hygiène.

L'invention concerne par ailleurs l'utilisation d'une composition selon l'invention, en tant que composition nettoyante.

La composition selon l'invention peut être utilisée dans tout type d'applications dans lesquelles il est usuel d'utiliser des compositions nettoyantes.

A titre d'exemple, la composition selon l'invention peut être utilisée en tant que composition nettoyante ou détergente, par exemple comme composition d'entretien ménager ou industriel, notamment comme composition de nettoyage pour surfaces dures ou comme produit vaisselle.

La composition selon l'invention peut également être utilisée dans le domaine pétrolier.

Avantageusement, la composition selon l'invention est utilisée en tant que produit d'hygiène ou en cosmétique, de préférence en tant que composition lavante et/ou démaquillante.

Cette composition peut être utilisée de sorte à la faire mousser avec de l'eau, ou peut être appliquée directement sur la peau sans addition d'eau.

En particulier, la composition selon l'invention selon son premier mode de réalisation, tel que décrit ci-avant, présente l'aspect d'un gel, et sera donc avantageusement utilisée de sorte à la faire mousser avec de l'eau.

Par exemple, l'utilisateur pourra appliquer de l'eau sur la partie de son corps à nettoyer, appliquer ensuite la composition selon l'invention sur cette partie, et enfin frotter de sorte à faire mousser ladite composition.

La composition selon l'invention selon son premier mode de réalisation pourra également être utilisée comme base pour un produit d'hygiène tel qu'une gelée micellaire.

La composition selon l'invention selon son second mode de réalisation, tel que décrit ci-avant, est sous la forme d'une solution, et sera donc avantageusement appliquée directement sur la peau, telle que la peau du visage, par exemple à l'aide d'un coton.

Par « solution », on entend un liquide de viscosité dynamique à 25°C inférieure à 40 mPa.s.

L'invention concerne en outre l'utilisation d'un concentré selon l'invention, en remplacement partiel ou total d'un tensioactif choisi parmi le groupe constitué par le laurylsulfate de sodium, le lauryl éther sulfate de sodium et/ou le cocamide diéthanolamine.

L'invention sera mieux comprise au vu des exemples qui suivent, donnés à titre illustratif, avec référence aux Figures suivantes :
- La Figure 1, qui est un diagramme représentant la stabilité au cours du temps de mousses formées à la surface de compositions préparées à partir de concentrés selon l'invention et à partir de composés sulfatés ou de MELs ;
- La Figure 2, qui comprend 2 photographies a et b illustrant la propriété nettoyante d'une composition selon l'invention et de compositions comparatives.

### Exemple 1 : Préparation de concentrés selon l'invention

### 1. Obtention de MELs

Les MELs ont été obtenus par un procédé de fermentation comprenant les étapes suivantes :
- la culture d'une souche de levure telle que *Pseudozyma aphidis* en présence d'huile végétale (colza) pour obtenir les MELs; et
- la récupération des MELs ainsi obtenus.

A l'issue de l'étape de récupération des MELs, un premier mélange de MELs (mélange de MELs 1A) est obtenu, qui présente les caractéristiques suivantes :
- Teneur en MELs: 55% en poids
- Teneur en autres composants : 45% en poids (dont 42% en poids d'acides gras libres et de triglycérides et 3% en poids d'eau et de souche),
les pourcentages en poids étant donnés par rapport au poids total du mélange de MELs obtenu.

Une étape de purification du mélange de MELs 1A a ensuite été réalisée par chromatographie d'adsorption sur colonne de silice, avec utilisation d'un mélange de solvants ayant un gradient de polarité croissant. Un second mélange de MELs (mélange de MELs 1B) a ainsi été obtenu, qui présente les caractéristiques suivantes :
- Teneur en MELs: au moins 98% en poids, par rapport au poids total du mélange de MELs obtenu.

En particulier, chacun des mélanges de MELs 1A et 1B comportent des MELs-A à une teneur de 52% en poids, des MELs-B à une teneur de 12% en poids, des MELs-C à une teneur de 35% en poids, et des MELs-D à une teneur de 1% en poids, les pourcentages en poids étant donnés par rapport au poids de la quantité totale de MELs.

### 2. Esters d'acide gras et de polyqlycérol

Du Radia® 7932 de chez OLEON a été utilisé. Ce produit est composé d'esters d'acide caprique et de polyglycérol-4 (polyglycerol-4 caprate ou polyglyceryl-4 caprate). Sa pureté en esters d'acide gras et de polyglycérol est supérieure à 95%.

Des esters d'acide oléique, d'acide caprique et d'acide isostéarique et de polyglycérol-10 ont été préparés selon le procédé d'estérification entre un acide gras (acide oléique, Radiacid 0215 de chez OLEON, acide caprique, Radiacid 610 de chez OLEON et acide isostéarique, Radiacid 0909 de chez OLEON) et du polyglycérol-10 de chez SPIGA NORD, dans un ratio molaire 1/1. L'acide gras et le polyglycérol sont mélangés en présence d'hydroxyde de calcium et chauffés à 220°C jusqu'à ce que l'indice d'acide soit inférieur à 1mgKOH/g.

### 3. Ester d'acide aras et de glycérol

Du Radia® 7907 de chez OLEON a été utilisé. Ce produit est composé d'esters d'acide caprylique et de glycérol (glycerol monocaprylate ou glyceryl monocaprylate).

### 4. Préparation de concentrés selon l'invention

Dans un récipient adapté, les composés sont mélangés manuellement, selon les formulations indiquées dans le Tableau 1 ci-dessous à une température de 60 °C, jusqu'à homogénéisation du concentré. La température ne doit préférablement pas excéder 60 °C.

Les différents concentrés préparés sont résumés dans le Tableau 1 suivant.

**Tableau 1 : Concentrés selon l'invention préparés dans l'Exemple 1**

| | Mélange de MELs 1B (%) | Radia® 7932 (%) | Radia® 7907 (%) | Oléate de PG-10 | Caprylate de PG-10 | Isostéarate de PG-10 |
|---|---|---|---|---|---|---|
| Concentré 1 | 25 | 75 | - | | | |
| Concentré 2 | 33,3 | 66,7 | - | | | |
| Concentré 3 | 50 | 50 | - | | | |
| Concentré 4 | 66,7 | 33,3 | | | | |
| Concentré 5 | 25 | 70,8 | 4,2 | | | |
| Concentré 6 | 33,3 | 62,9 | 3,8 | | | |
| Concentré 7 | 50 | 47,2 | 2,8 | | | |
| Concentré 8 | 66,7 | 31,44 | 1,86 | | | |
| Concentré 15 | 50 | | 2,8 | 47,2 | | |
| Concentré 16 | 50 | | 2,8 | | 47,2 | |
| Concentré 17 | 50 | | 2,8 | | | 47,2 |

| | | | | | | |
|---|---|---|---|---|---|---|
| *Les pourcentages indiques sont des pourcentages en poids sur le poids total de concentré. | | | | | | |

### Exemple 2 : Evaluation de la propriété épaississante des concentrés selon l'invention et de concentrés comparatifs

La propriété épaississante des concentrés selon l'invention et de concentrés comparatifs a été évaluée.

### 1. Matériel et Méthodes

### 1.1. Matériel

Les produits suivants ont été utilisés :
- les concentrés 1 à 8 préparés à l'Exemple 1
- le mélange de MELs 1B préparé à l'Exemple 1
- du Radia® 7932 (OLEON)
- du Radia® 7907 (OLEON)
- de l'eau déminéralisée.

Le matériel suivant a été utilisé :
- des flacons en verre
- une spatule
- un rhéomètre (TA instruments AR 2000).

### 1.2. Méthodes

### Concentrés selon l'invention

Les concentrés 1 à 8 préparés à l'Exemple 1 ont été utilisés.

### Préparation des concentrés comparatifs

Dans un récipient adapté, les différents composés sont mélangés manuellement, à une température de 60°C, jusqu'à homogénéisation du concentré. Lorsque des MELs sont utilisés dans la préparation du concentré, la température ne doit préférablement pas excéder 60 °C.

Les différents concentrés comparatifs préparés sont résumés dans le Tableau 2 suivant.

**Tableau 2 : Concentrés comparatifs préparés dans l'Exemple 2**

| | Mélange de MELs 1B (%) | Radia® 7932 (%) | Radia® 7907 (%) |
|---|---|---|---|
| Concentré 9 comparatif | 0 | 100 | - |
| Concentré 10 comparatif | 10 | 90 | - |
| Concentré 11 comparatif | 100 | 0 | - |
| Concentré 12 comparatif | 0 | 94,4 | 5,6 |
| Concentré 13 comparatif | 10 | 84,96 | 5,04 |
| Concentré 14 comparatif | 15 | 80,24 | 4,76 |

| | | | |
|---|---|---|---|
| *Les pourcentages indiqués sont des pourcentages en poids sur le poids total de concentré. | | | |

### Evaluation de la propriété épaississante des concentrés 1 à 14

Dans des flacons en verre, 10% en poids des concentrés 1 à 8 selon l'invention et des concentrés 9 à 14 comparatifs ont été respectivement ajoutés à 90% en poids d'eau, les % en poids étant indiqués par rapport au poids total de chaque composition obtenue. L'ajout de l'eau dans les flacons comprenant les différents concentrés se fait sous agitation manuelle avec une spatule.

La viscosité dynamique des compositions 1 à 14 a été évaluée, à l'aide du rhéomètre, à une température de 25°C et à une vitesse de 10 tr/min.

La viscosité dynamique de l'eau (contrôle) est de 1 mPA.s.

L'aspect des différentes compositions a également été évalué à l'œil nu.

Les résultats sont présentés dans le Tableau 3 ci-dessous.

**Tableau 3 : Viscosité dynamique et aspect des compositions 1 à 14 préparées dans l'Exemple 2**

| | | Viscosité (mPa.s) | Aspect |
|---|---|---|---|
| Composition 1 selon l'invention | Concentré 1 selon l'invention + eau | 44 | Aspect gel en partie, une phase |
| Composition 2 selon l'invention | Concentré 2 selon l'invention + eau | 48 | Aspect gel en partie, une phase |
| Composition 3 selon l'invention | Concentré 3 selon l'invention + eau | 586 | Aspect gel, une phase |
| Composition 4 selon l'invention | Concentré 4 selon l'invention + eau | 980 | Aspect gel, une phase |
| Composition 5 selon l'invention | Concentré 5 selon l'invention + eau | 56 | Aspect gel, une phase |
| Composition 6 selon l'invention | Concentré 6 selon l'invention + eau | 400 | Aspect gel, une phase |
| Composition 7 selon l'invention | Concentré 7 selon l'invention + eau | 500 | Aspect gel, une phase |
| Composition 8 selon l'invention | Concentré 8 selon l'invention + eau | 70 | Aspect gel, une phase |
| Composition 9 comparative | Concentré 9 comparatif + eau | 5 | Aspect eau, clair |
| Composition 10 comparative | Concentré 10 comparatif + eau | 13 | Deux phases |
| Composition 11 comparative | Concentré 11 comparatif + eau | 5 | Deux phases |
| Composition 12 comparative | Concentré 12 comparatif + eau | 5,5 | Aspect eau, clair |
| Composition 13 comparative | Concentré 13 comparatif + eau | 4,5 | 1 phase, trouble, translucide |
| Composition 14 comparative | Concentré 14 comparatif + eau | <5 | 1 phase, trouble, translucide |

Les résultats montrent que les compositions 1 à 8 comprenant un concentré selon l'invention et de l'eau ont une viscosité dynamique supérieure à celle de l'eau pure, et présentent en outre l'aspect d'un gel. Au contraire, les compositions 9 à 14 comprenant des concentrés comparatifs ont une viscosité proche de celle de l'eau et ne présentent pas l'aspect d'un gel.

Un concentré selon l'invention permet d'augmenter la viscosité de l'eau. On entend ici qu'une composition comprenant un concentré selon l'invention et de l'eau aura une viscosité supérieure à celle de l'eau seule.

Le concentré selon l'invention possède donc une bonne propriété épaississante, et peut donc être utilisé en tant qu'agent épaississant.

En outre, un concentré selon l'invention permet de conférer à l'eau l'aspect d'un gel.

### Exemple 3 : Effet de la quantité de concentré sur la viscosité de l'eau

Des compositions comprenant différentes quantités du concentré 2 selon l'invention préparé à l'Exemple 1 et de l'eau ont été préparées, selon la méthode décrite dans l'Exemple 2.

Des mesures de viscosités ont été effectuées, de la même manière que dans l'Exemple 2.

Les résultats sont indiqués dans le Tableau 4 ci-dessous.

**Tableau 4 : Effet de la quantité de concentré sur la viscosité de l'eau**

| Quantité de concentré (%) | Quantité d'eau (%) | Viscosité (mPa.s) |
|---|---|---|
| 1 | 99 | 7 |
| 3 | 97 | 9 |
| 5 | 95 | 18 |
| 10 | 90 | 48 |
| 15 | 85 | 141 |
| 20 | 80 | 175 |
| 30 | 70 | 981 |
| 50 | 50 | 4380 |

| | | |
|---|---|---|
| *Les pourcentages indiqués sont des pourcentages en poids sur le poids total de la composition. | | |

Les résultats montrent que des quantités croissantes de concentrés selon l'invention permettent d'augmenter la viscosité de l'eau.

### Exemple 4 : Evaluation de la propriété moussante d'un concentré selon l'invention et de concentrés comparatifs - Evaluation de la stabilité des mousses obtenues

### 1. Matériel et Méthodes

### 1.1. Matériel

Les produits suivants ont été utilisés :
- le concentré 7 selon l'invention préparé dans l'Exemple 1
- les concentrés 15 et 16 préparés dans l'Exemple 1
- le concentré 11 comparatif préparé dans l'Exemple 2
- SLES
de l'eau.

Le matériel suivant a été utilisé :
- bain-marie,
- débitmètre,
- un dispositif d'écoulement de l'air à 94mL/min.

### 1.2. Méthodes

Le protocole mis en œuvre est basé sur celui décrit dans la norme ASTM D892.

Dans des tubes à essais, 0,02%, 0,2% et 2% en poids du concentré 7, ainsi que respectivement 99,98% 99,8% et 98% en poids d'eau ont été ajoutés pour obtenir 3 compositions à tester. Dans deux autres tubes, respectivement 2% en poids de concentré 15 et 16 ont été introduits ainsi que 98% en poids d'eau.

Les tubes à essais sont ensuite mis dans un bain à température contrôlée. Après 15 minutes, la température souhaitée de 25°C est atteinte.

De l'air est ensuite pompé à travers une pierre sphérique et poreuse dans chaque composition à tester. On crée ainsi de petites bulles d'air qui forment une dispersion d'air dans l'eau. Une mousse se forme si les bulles de gaz montent à la surface et si elles ne se brisent pas avant. Les bulles remplies de gaz possèdent des parois en fine lamelle liquide. Les compositions à tester sont maintenues à une température de 25 °C, et sont soumises au pompage d'air pendant 5 minutes. Le débit d'air est ensuite arrêté.

Le volume de mousse formée en surface de chacune des compositions obtenues à partir du concentré 7, du concentré 15 et du concentré 16 a été évalué, directement après l'arrêt du débit d'air.

Le temps nécessaire pour que la mousse se désagrège est observé pour les compositions comprenant 0,2% et 2% en poids du concentré 7, ainsi que pour les compositions comprenant respectivement 2% en poids du concentré 15 et 2% en poids du concentré 16. Plus particulièrement, la stabilité de la mousse en surface de cette composition a été évaluée en mesurant le volume de mousse en fonction du temps. Plus précisément, le volume de mousse a été évalué pendant 10 minutes après sa formation, à des intervalles de temps de 60 secondes.

Durant tout le temps nécessaire aux mesures, les compositions à tester sont maintenues à une température de 25 °C.

Un test identique a été réalisé pour des compositions à tester comprenant 0,2% en poids de concentré 11 comparatif (MELs),0,2% et 0,5% en poids de lauryl éther sulfate de sodium (SLES) et respectivement 99,8%, 99,8% et 99,5% en poids d'eau, par rapport au poids total de composition. Le SLES est utilisé comme référence. Le SLES est un agent tensioactif ayant de très bonnes propriétés moussante et détergente (nettoyante).

### 2. Résultats

### Propriété moussante

Les résultats sont présentés dans le Tableau 5 ci-dessous.

**Tableau 5 : Propriété moussante des concentrés 7, 15 et 16 selon l'invention et du concentré 11 comparatif et du SLES**

| Concentré | Quantité de concentré (%) | Quantité d'eau (%) | Volume de mousse formé (mL) |
|---|---|---|---|
| 7 | 0,02 | 99,98 | 210 |
| | 0,2 | 99,8 | 500 |
| | 2 | 98 | 700 |
| 15 | 2 | 98 | 760 |
| 16 | 2 | 98 | 700 |
| 11 | 0,2 | 99,8 | 600 |
| SLES | 0,2 | 99,8 | 500 |
| SLES | 0,5 | 99,5 | 700 |

Ces résultats montrent que les volumes de mousse formés en surface des compositions comprenant les concentrés 7, 15 ou 16 selon l'invention sont élevés.

Un concentré selon l'invention a donc une très bonne propriété moussante. On entend ici qu'un concentré selon l'invention, lorsqu'il est mis au contact avec de l'eau, permet la formation d'une mousse de volume élevé à la surface de la composition obtenue.

En outre, la composition à tester comprenant 0,2% en poids du concentré 7 selon l'invention a permis l'obtention d'une mousse de volume similaire à une mousse obtenue avec une composition comprenant 0,2% en poids de lauryl éther sulfate de sodium et 99,8% en poids d'eau par rapport au poids total de la composition.

Un concentré selon l'invention est une bonne solution de remplacement du lauryl éther sulfate de sodium, ou du laurylsulfate de sodium.

### Stabilité des mousses

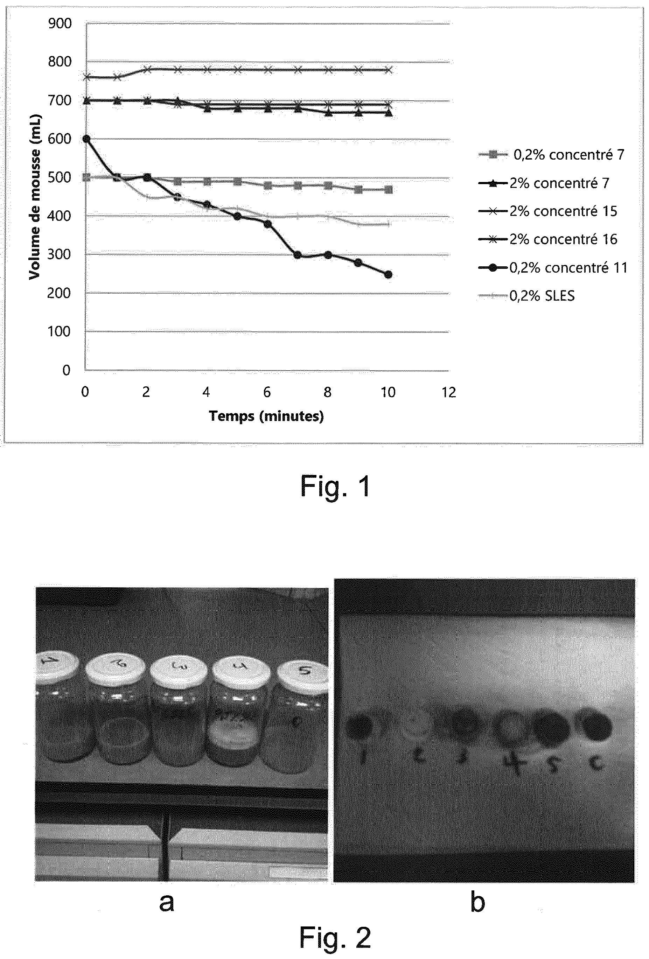

Les résultats sont présentés à la Figure 1.

Ces résultats montrent que les mousses obtenues avec les concentrés 7, 15 et 16 selon l'invention étaient stables pendant les 10 minutes du test, c'est-à-dire que le volume de ces mousses n'a pas ou a très peu diminué pendant 10 minutes.

Les mousses obtenues avec le SLES, en particulier celle obtenue à partir de la composition comprenant 0,2% en poids de SLES, étaient moins stables pendant les 10 minutes du test, une diminution du volume de ces mousses étant visible sur la Figure 1.

Ainsi, la mousse formée à la surface d'une composition comprenant un concentré selon l'invention est stable. Par « stable », on entend que le volume de mousse formée ne diminue pas ou diminue très peu au cours du temps, c'est-à-dire de moins de 50 mL, pendant 10min, préférentiellement de moins de 25mL pendant 10 min.

La composition comportant 0,2% en poids de concentré 11 comparatif forme certes un volume de mousse élevé mais ce volume n'est pas stable et chute rapidement (-100 mL en 1 minute, -350mL en 10 minutes), comme cela est visible sur la Figure 1.

### Exemple 5 : Evaluation de la propriété tensioactive de concentrés selon l'invention et de concentrés comparatifs

### 1. Matériel et Méthodes

### 1.1. Matériel

- Les concentrés 3 et 7 selon l'invention préparés dans l'Exemple 1
- De l'eau pure
- le tensiomètre K100 (KRUSS)

### 1.2. Méthodes

### Tensions de surface

Les concentrés 3 et 7 selon l'invention ont été ajoutés à différentes concentrations dans l'eau pure et des mesures de tensions de surface ont été réalisées.

La tension de surface a été mesurée à l'aide du tensiomètre, grâce à la méthode de la lame de Wilhelmy.

La tension de surface de l'eau pure a également été mesurée. Celle-ci est de 71,4 mN/m.

Les résultats sont indiqués dans le Tableau 6 ci-dessous.

**Tableau 6 : Tensions de surface**

| Concentré | Quantité de concentré dans l'eau (%) | Tension de surface (mN/m) |
|---|---|---|
| 3 | 0,02 | 27,6 |
| 3 | 0,2 | 27,6 |
| 3 | 2 | 27,3 |

| | | |
|---|---|---|
| *Les pourcentages indiqués sont des pourcentages en poids sur le poids total de la composition. | | |

Les résultats présentés dans le Tableau 6 montrent notamment qu'un concentré selon l'invention permet de diminuer la tension de surface de l'eau. Un concentré selon l'invention peut donc par exemple être utilisé dans des applications de nettoyage.

### Tensions interfaciales

Les concentrés 3 et 7 selon l'invention puis de l'huile minérale ont été ajoutés à différentes concentrations à de l'eau pure, et des mesures de tensions interfaciales ont été réalisées.

La tension interfaciale d'une préparation eau/huile minérale a également été mesurée. Celle-ci est d'environ 43 mN/m.

Les résultats sont indiqués dans le Tableau 7 ci-dessous.

**Tableau 7 : Tensions interfaciales**

| Concentré | Quantité de concentré dans l'eau (%) | Tension interfaciale (mN/m) |
|---|---|---|
| 3 | 0,02 | 1,1 |
| 3 | 0,2 | 0,6 |
| 3 | 2 | 0,2 |
| 7 | 0,02 | 1,1 |
| 7 | 0,2 | 0,6 |
| 7 | 2 | 0,2 |

| | | |
|---|---|---|
| *Les pourcentages indiqués sont des pourcentages en poids sur le poids total de la composition. | | |

Les valeurs de tensions interfaciales obtenues avec les concentrés selon l'invention sont suffisamment basses pour qu'un concentré selon l'invention ait la capacité de disperser une huile minérale dans l'eau. Un concentré selon l'invention peut donc par exemple être utilisé dans des applications de nettoyage.

### Exemple 6 : Propriété nettoyante d'une composition selon l'invention -

### Application en cosmétique

### 1. Matériel et Méthodes

### 1.1. Matériel

Les produits suivants ont été utilisés :
- la composition 7 selon l'invention préparée à l'Exemple 2
- la composition 3 selon l'invention préparée à l'Exemple 2
- du laurylsulfate de sodium (VWR®, 100% pur)
- de l'eau.

Le matériel suivant a été utilisé :
- des pots en verre avec leur couvercle,
- des bouchons blancs de bouteilles de 15 mL
- du maquillage (fond de teint, True Match™, Super Blendable Makeup, L'OREAL®).

### 1.2. Méthodes

Dans un pot en verre, de l'eau ainsi que la composition à tester sont ajoutés.

Un bouchon blanc est ensuite recouvert de maquillage, puis plongé dans le pot en verre. Le pot en verre est fermé avec son couvercle puis soumis à agitation à 244 rpm pendant 60 minutes.

Au bout de 60 minutes, la proportion de maquillage qui a été éliminée du bouchon et transférée dans le mélange eau/composition à tester est mesurée.

Le pourcentage de maquillage éliminé est calculé selon la formule suivante :
100 - (poids de maquillage sur le bouchon avant l'expérience - poids de maquillage sur le bouchon après l'expérience) x 100 / poids de maquillage sur le bouchon avant l'expérience.

Dans l'expérience 1 ci-après, cette méthode a été mise en oeuvre pour évaluer la propriété démaquillante de la composition 7 selon l'invention préparée à l'Exemple 2 et de compositions comparatives. Les compositions comparatives comprennent du SLS. Le SLS, tout comme le SLES, est un agent tensioactif ayant de très bonnes propriétés moussante et détergente (nettoyante).

Dans l'expérience 2 ci-après, cette méthode a été mise en oeuvre pour évaluer la propriété démaquillante de la composition 3 selon l'invention préparée à l'Exemple 2.

### Expérience 1 :

Le détail des tests réalisés et les résultats sont présentés dans le Tableau 8 ci-dessous.

**Tableau 8 : Tests et résultats de l'expérience 1**

| **Test** | **Composition à tester** | **% de maquillage éliminé** |
|---|---|---|
| **Test 1** | 1 g de composition 7 selon l'invention dans 49 g d'eau | 95,92 |
| **Test 2** | 5 g de composition 7 selon l'invention dans 49 g d'eau | 99,07 |
| **Test 3 comparatif** | 0,05 g de laurylsulfate de sodium dans 50 g d'eau | 90,70 |
| **Test 4 comparatif** | 0,25 g de laurylsulfate de sodium dans 50 g d'eau | 98,37 |
| **Test 5 (contrôle)** | Eau seule | 71,72 |

Les résultats de l'expérience 1 sont également présentés à la Figure 2 (comprenant les photographies a et b).

Il peut être constaté sur la photographie a que la proportion de maquillage qui a été éliminée du bouchon et transférée dans le mélange eau/composition 7 selon l'invention (pots en verre marqués 1 et 2) est supérieure à celle qui a été éliminée des bouchons et transférée dans les mélanges eau/composition comparative (pots en verre marqués 3 et 4) et dans l'eau seule (pot en verre marqué 5).

Cela ressort également de la photographie b, qui montre la quantité de maquillage qui a été éliminée des bouchons à la fin de chaque test 1 à 5.

Le bouchon numéroté 0 sur la photographie b correspond à un bouchon contrôle avant insertion dans un pot en verre.

Ces résultats montrent que la composition 7 selon l'invention a une propriété démaquillante équivalente voir même supérieure à celle des compositions comparatives à base laurylsulfate de sodium.

### Expérience 2 :

Le détail des tests réalisés et les résultats sont présentés dans le Tableau 9 ci-dessous.

**Tableau 9 : Tests et résultats de l'expérience 2**

| **Test** | **Composition à tester** | **% de maquillage éliminé** |
|---|---|---|
| **Test 6** | 1 g de composition 3 selon l'invention dans 49 g d'eau | 90,99 |
| **Test 7** | 5 g de composition 3 selon l'invention dans 49 g d'eau | 97,69 |
| **Test 8 (contrôle)** | Eau seule | 70,76 |

Il peut être constaté que la proportion de maquillage qui a été éliminée du bouchon et transférée dans le mélange eau/composition 3 selon l'invention (tests 6 et 7) est notablement supérieure à celle qui a été éliminée des bouchons et transférée dans l'eau seule (test 8).

### Exemple 7 : Remplacement de cocamide diéthanolamine (cocamide DEA) et/ou de laurvl éther sulfate de sodium (SLES) par un concentré selon l'invention

Le concentré 7 selon l'invention a été utilisé dans la préparation de compositions nettoyantes, de type détergent pour vaisselle ou shampoing, en remplacement de cocamide DEA et/ou de SLES.

Le cocamide DEA est un agent tensioactif ayant de bonnes propriétés moussante et épaississante.

La viscosité des compositions préparées après un jour a été évaluée selon la méthode décrite dans l'Exemple 2.

Le volume de mousse formé à la surface des différentes compositions a été évalué selon la méthode décrite dans l'Exemple 4.

Le détail des compositions nettoyantes préparées et les résultats des différentes mesures sont indiqués dans le Tableau 10 ci-dessous. Les compositions nettoyantes sont préparées par simple mélange de leurs composants.

**Tableau 10: Compositions préparées et résultats des tests 9 à 16 de l'Exemple 7**

| | **Test 9** | **Test 10** | **Test 11** | **Test 12** | **Test 13** | **Test 14** | **Test 15** | **Test 16** |
|---|---|---|---|---|---|---|---|---|
| | %* | | | | | | | |
| **Euramid V (cocamide DEA)** | 3 | | | | | | | |
| **SLES (28% actif)** | 45 | 45 | 40 | 30 | 20 | 10 | 5 | 0 |
| **Concentré 7 selon l'invention** | 0 | 3 | 8 | 18 | 28 | 38 | 43 | 48 |
| **Benzoate de sodium** | 0,5 | 0,5 | 0,5 | 0,5 | 0,5 | 0,5 | 0,5 | 0,5 |
| **NaCl** | 0 ou 2 | 0 ou 2 | 0 ou 2 | 0 ou 2 | 0 ou 2 | 0 ou 2 | 0 ou 2 | 0 ou 2 |
| **Eau** | qsp 100 | qsp 100 | qsp 100 | qsp 100 | qsp 100 | qsp 100 | qsp 100 | qsp 100 |
| **pH** | 5-6 | 5-6 | 5-6 | 5-6 | 5-6 | 5-6 | 5-6 | |
| **Viscosité après 1 jour (sans NaCl)** | 40 | 10 | 120 | 6100 | 7500 | 24500 | 29000 | 25000 |
| **Viscosité après 1 jour (avec NaCl)** | 6300 | 2020 | 1320 | 4100 | 8600 | 15200 | 17700 | 20900 |
| **Apparence** | claire | claire | claire | Mousse ,trouble | Mousse ,trouble | Mousse claire | Mousse ,trouble | Mousse ,trouble |
| **Mousse après 1 minute (avec NaCl)** | 850 | 760 | 780 | 770 | 800 | 900 | 780 | 850 |
| **Mousse après 10 minutes (avec NaCl)** | 860 | 760 | 780 | 770 | 800 | 880 | 760 | 830 |

| | | | | | | | | |
|---|---|---|---|---|---|---|---|---|
| *Les pourcentages indiqués sont des pourcentages en poids sur le poids total de la composition | | | | | | | | |

Les résultats montrent que le remplacement de SLES par des quantités croissantes du concentré selon l'invention résulte en une augmentation de la viscosité. Cette augmentation de la viscosité est obtenue malgré l'absence de cocamide DEA et de NaCl.

Le volume des mousses formées à la surface des différentes compositions est par ailleurs élevé.

Un concentré selon l'invention est donc une bonne solution de remplacement du cocamide DEA, du lauryl éther sulfate de sodium, et/ou du laurylsulfate de sodium.

Plus particulièrement, une composition détergente préparée à partir d'un concentré selon l'invention aura à la fois une viscosité élevée et une bonne propriété moussante.

Les concentrés 15 et 17 selon l'invention ont également été utilisés dans la préparation de compositions nettoyantes, de type détergent pour vaisselle ou shampoing, en remplacement de cocamide DEA et/ou de SLES (tests 17 et 18 respectivement).

Le pH de ces compositions a été ajusté à 5,8 en ajoutant de l'acide citrique.

La viscosité des compositions préparées après un jour a été évaluée selon la méthode décrite dans l'Exemple 2.

Le volume de mousse formé à la surface des différentes compositions a été évalué selon la méthode décrite dans l'Exemple 4.

Le détail des compositions nettoyantes préparées et les résultats des différentes mesures sont indiqués dans le Tableau 11 ci-dessous. Les compositions nettoyantes sont préparées par simple mélange de leurs composants.

**Tableau 11 : Compositions préparées et résultats des tests 17 et 18 de l'Exemple 7**

| | **Test 17** | **Test 18** |
|---|---|---|
| | %* | |
| **Euramid V (cocamide DEA)** | 3 | 3 |
| **SLES (28% actif)** | 20 | 20 |
| **Concentré 15 selon l'invention** | 28 | |
| **Concentré 17 selon l'invention** | | 28 |
| **Benzoate de sodium** | 0,5 | 0,5 |
| **NaCl** | 0 | 0 |
| **Eau** | qsp 100 | qsp 100 |
| **pH** | 5,8 | 5,8 |
| **Viscosité après 1 jour (sans NaCl)** | 7230 | 9032 |
| **Apparence** | trouble | trouble |
| **Mousse après 10 minutes** | 770 | 820 |

| | | |
|---|---|---|
| *Les pourcentages indiqués sont des pourcentages en poids sur le poids total de la composition | | |

Les résultats montrent à nouveau que les concentrés selon l'invention sont une bonne solution de remplacement de SLES.

Comparativement au test 13 avec le concentré 7, les tests 17 et 18 montrent que les concentrés 15 et 17 permettent également une augmentation de viscosité, en particulier en l'absence de NaCl.

Le volume des mousses formées à la surface des différentes compositions est par ailleurs élevé.

Une composition nettoyante, tel qu'un shampoing, préparée à partir d'un concentré selon l'invention aura à la fois une viscosité élevée et une bonne propriété moussante.

## Revendications

1. Concentré comprenant :
- au moins 20% en poids d'au moins un lipide de mannosylérythritol, (MEL) par rapport au poids total du concentré, et
- au moins 30% en poids d'au moins un ester d'acide gras et de polyglycérol de HLB supérieure ou égale à 9, par rapport au poids total du concentré dans lequel le ratio en poids lipide(s) de mannosylérythritol/ester(s) d'acide(s) gras et de polyglycérol de HLB supérieure ou égale à 9 est compris dans la gamme [1 / 3;3/1].

2. Concentré selon la revendication 1, dans lequel le ratio en poids lipide(s) de mannosylérythritol/ester(s) d'acide(s) gras et de polyglycérol de HLB supérieure ou égale à 9 est compris dans la gamme [1 / 2 ; 2 / 1].

3. Concentré selon la revendication 1 ou 2, comprenant au moins deux MELs choisis parmi le groupe constitué par les MELs-A, MELs-B, MELs-C et MELs-D.

4. Concentré selon l'une quelconque des revendications 1 à 3, comprenant en outre au moins un ester d'acide gras et de glycérol.

5. Concentré selon la revendication 4, dans lequel le au moins un ester d'acide gras et de glycérol est un ester d'acide caprylique et de glycérol.

6. Composition comprenant :
- au moins un MEL,
- au moins un ester d'acide gras et de polyglycérol de HLB supérieure ou égale à 9, et
- de l'eau
dans laquelle le ratio en poids lipide(s) de mannosylérythritol/ester(s) d'acide(s) gras et de polyglycérol est compris dans la gamme [1 / 3 ; 3 / 1], et
dans laquelle la quantité totale de MEL(s) et d'ester(s) d'acide(s) gras et de polyglycérol est comprise entre 3% et 75% en poids, sur le poids total de la composition.

7. Composition sous la forme d'une solution, comprenant :
- au moins un MEL,
- au moins un ester d'acide gras et de polyglycérol de HLB supérieure ou égale à 9, et
- de l'eau
dans laquelle le ratio en poids lipide(s) de mannosylérythritol/ester(s) d'acide(s) gras et de polyglycérol est compris dans la gamme [1 / 3 ; 3 / 1], et
dans laquelle la quantité totale de MEL(s) et d'ester(s) d'acide(s) gras et de polyglycérol exprimée en pourcentage en poids sur le poids total de la composition est comprise dans la gamme [0,05 ; 3[.

8. Utilisation d'un concentré selon l'une quelconque des revendications 1 à 5, en tant qu'agent épaississant, moussant et/ou nettoyant.

9. Utilisation d'une composition selon la revendication 6 ou 7, en tant que composition nettoyante.

10. Utilisation d'un concentré selon l'une quelconques des revendications 1 à 5, en remplacement partiel ou total d'un tensioactif choisi parmi le groupe constitué par le laurylsulfate de sodium, le lauryl éther sulfate de sodium et/ou le cocamide diéthanolamine.

## Patentansprüche

1. Konzentrat, umfassend:
- mindestens 20 Gew.-% mindestens eines Mannosylerythritol-Lipids (MEL), bezogen auf das Gesamtgewicht des Konzentrats, und
- mindestens 30 Gew.-% mindestens eines Esters von Fettsäure und Polyglycerin mit einem HLB, das größer als oder gleich 9 ist, bezogen auf das Gesamtgewicht des Konzentrats,
wobei das Gewichtsverhältnis von Mannosylerythritol-Lipid(en)/Ester(n) von Fettsäure(n) und Polyglycerin mit einem HLB, das größer als oder gleich 9 ist, im Bereich von [1/3; 3/1] liegt.

2. Konzentrat nach Anspruch 1, wobei das Gewichtsverhältnis von Mannosylerythritol-Lipid(en)/Ester(n) von Fettsäure(n) und Polyglycerin mit einem HLB, das größer als oder gleich 9 ist, im Bereich von [1/2; 2/1] liegt.

3. Konzentrat nach Anspruch 1 oder 2, umfassend mindestens zwei MELs, die aus der Gruppe bestehend aus MELs-A, MELs-B, MELs-C und MELs-D ausgewählt sind.

4. Konzentrat nach einem der Ansprüche 1 bis 3, weiterhin umfassend mindestens einen Ester von Fettsäure und Glycerin.

5. Konzentrat nach Anspruch 4, wobei der mindestens eine Ester von Fettsäure und Glycerin ein Ester von Caprylsäure und Glycerin ist.

6. Zusammensetzung, umfassend:
- mindestens ein MEL,
- mindestens einen Ester von Fettsäure und Polyglycerin mit einem HLB, das größer als oder gleich 9 ist, und
- Wasser,
wobei das Gewichtsverhältnis von Mannosylerythritol-Lipid(en)/Ester(n) von Fettsäure(n) und Polyglycerin im Bereich von [1/3; 3/1] liegt, und
wobei die Gesamtmenge von MEL(s) und Ester(n) von Fettsäure(n) und Polyglycerin zwischen 3 Gew.-% und 75 Gew.-% auf das Gesamtgewicht der Zusammensetzung liegt.

7. Zusammensetzung in der Form einer Lösung, umfassend:
- mindestens ein MEL,
- mindestens einen Ester von Fettsäure und Polyglycerin mit einem HLB, das größer als oder gleich 9 ist, und
- Wasser,
wobei das Gewichtsverhältnis von Mannosylerythritol-Lipid(en)/Ester(n) von Fettsäure(n) und Polyglycerin im Bereich von [1/3; 3/1] liegt, und
wobei die Gesamtmenge von MEL(s) und Ester(n) von Fettsäure(n) und Polyglycerin, als Gewichtsprozentanteil auf das Gesamtgewicht der Zusammensetzung ausgedrückt, im Bereich [0,05; 3[ liegt.

8. Verwendung eines Konzentrats nach einem der Ansprüche 1 bis 5 als ein Verdickungs-, Schäumungs- und/oder Reinigungsmittel.

9. Verwendung einer Zusammensetzung nach Anspruch 6 oder 7 als Reinigungszusammensetzung.

10. Verwendung eines Konzentrats nach einem der Ansprüche 1 bis 5 zum partiellen oder vollständigen Ersatz eines Tensids, das aus der Gruppe bestehend aus Natriumlaurylsulfat, Natriumlaurylethersulfat und/oder Cocamiddiethanolamin ausgewählt ist.

## Claims

1. Concentrate comprising:
- at least 20% by weight of at least one mannosylerythritol lipid (MEL), with respect to the total weight of the concentrate, and
- at least 30% by weight of at least one polyglycerol fatty acid ester having an HLB greater than or equal to 9, with respect to the total weight of the concentrate,
in which the ratio by weight of mannosylerythritol lipid(s)/polyglycerol fatty acid ester(s) having an HLB greater than or equal to 9 is comprised within the range [1/3; 3/1].

2. Concentrate according to claim 1, in which the ratio of mannosylerythritol lipid(s)/polyglycerol fatty acid ester(s) having an HLB greater than or equal to 9 is comprised within the range [1/2; 2/1].

3. Concentrate according to claim 1 or 2, comprising at least two MELs selected from the group constituted by the MELs-A, MELs-B, MELs-C and MELs-D.

4. Concentrate according to any one of claims 1 to 3, also comprising at least one glycerol fatty acid ester.

5. Concentrate according to claim 4, in which the at least one glycerol fatty acid ester is a glycerol caprylic acid ester.

6. Composition comprising:
- at least one MEL,
- at least one polyglycerol fatty acid ester having an HLB greater than or equal to 9, and
- water
in which the weight ratio of mannosylerythritol lipid(s)/polyglycerol fatty acid ester(s) is comprised within the range [1/3; 3/1], and
in which the total quantity of quantity of MEL(s) and polyglycerol fatty acid ester(s) is comprised between 3% and 75% by weight, of the total weight of the composition.

7. Composition in the form of a solution, comprising:
- at least one MEL,
- at least one polyglycerol fatty acid ester having an HLB greater than or equal to 9, and
- water
in which the weight ratio of mannosylerythritol lipid(s)/polyglycerol fatty acid ester(s) is comprised within the range [1/3; 3/1], and
in which the total quantity of MEL(s) and polyglycerol fatty acid ester(s) expressed as a percentage by weight of the total weight of the composition is comprised within the range [0.05; 3[.

8. Use of a concentrate according to any one of claims 1 to 5 as a thickening, foaming and/or cleaning agent.

9. Use of a composition according to claim 6 or 7 as a cleaning composition.

10. Use of a concentrate according to any one of claims 1 to 5, for partially or totally replacing a surfactant selected from the group constituted by sodium lauryl sulphate, sodium lauryl ether sulphate and/or cocamide diethanolamine.
